# DEMANDE DE BREVET EUROPEEN

(11) **EP 1 837 012 A1**
(43) Date de publication de la demande: **26.09.2007**
(21) Numéro de dépôt: 07101061.5
(22) Date de dépôt: 24.01.2007
(51) Int. Cl.: A61K 8/36, A61K 8/34, A61Q 19/10, A61Q 1/14

(54) **Composition cosmétique moussante**

(30) Priorité: 13.03.2006 FR 0650834
(71) Demandeur: L'Oréal, 75008 Paris (FR)
(72) Inventeur: Aubrun-Sonneville, Odile, 92160, ANTONY (FR)
(74) Mandataire: Rasson, Catherine

(57) **Abrégé**

La présente demande concerne une composition moussante pour application topique, contenant dans un milieu aqueux, un ou plusieurs sels d'acide gras en une quantité d'au moins 20 % du poids total de la composition, et au moins un tensioactif non ionique choisi parmi les dérivés d'alcool béhénylique ou d'acide béhénique.

Ces compositions peuvent se présenter sous forme de crèmes ou de laits, et elles présentent une très bonne stabilité même après chauffage à température très élevée. Elles peuvent être utilisées notamment dans les domaines cosmétique ou dermatologique, comme produits de nettoyage ou de démaquillage de la peau, du cuir chevelu et/ou des cheveux.

## Description

La présente invention se rapporte à une composition moussante rinçable à base de savon, ainsi qu'à son utilisation dans les domaines cosmétique ou dermatologique, notamment comme produits de nettoyage ou de démaquillage de la peau, du cuir chevelu et/ou des cheveux.

Le nettoyage de la peau est très important pour le soin du visage. Il doit être le plus performant possible car les résidus gras tels que l'excès de sébum, les restes des produits cosmétiques utilisés quotidiennement et les produits de maquillage notamment les produits « waterproof » résistants à l'eau, s'accumulent dans les replis cutanés et peuvent obstruer les pores de la peau et entraîner l'apparition de boutons.

On connaît plusieurs grands types de produits de nettoyage de la peau : les lotions et gels aqueux détergents moussants, les huiles et gels anhydres nettoyants rinçables, et les compositions moussantes, notamment les crèmes moussantes.

Les huiles et gels anhydres rinçables ont une action nettoyante grâce aux huiles contenues dans ces formulations. Ces huiles permettent la solubilisation des résidus gras et la dispersion des pigments de maquillage. Ces produits sont efficaces et bien tolérés. Toutefois, ils présentent l'inconvénient d'être lourds, de ne pas mousser et de ne pas conférer de sensation de fraîcheur à l'application, ce qui est pénalisant d'un point de vue cosmétique.

Par ailleurs, les lotions et gels aqueux détergents moussants ont une action nettoyante grâce aux tensioactifs qui mettent en suspension les résidus gras et les pigments des produits de maquillage. Ils sont efficaces et agréables à utiliser du fait qu'ils moussent et qu'ils sont facilement éliminés. Toutefois, les lotions sont généralement assez fluides, ce qui rend leur manipulation parfois délicate, et il est difficile d'épaissir les gels tout en gardant de bonnes propriétés moussantes.

Les compositions moussantes contenant des savons qui sont des sels d'acides gras, sont connues pour avoir de bonnes performances moussantes et donner un bon nettoyage. Ces compositions peuvent être sous forme liquide comme décrit par exemple dans le document EP-A-339994, ou sous forme de crème comme décrit par exemple dans les documents EP-A-1,166,747 et EP-1,486,558.

L'utilisation de crèmes est préférée par le consommateur car les crèmes, contrairement aux liquides, ne coulent pas lors de l'application, et elles donnent un meilleur confort sur la peau après utilisation. Ainsi, le document EP-A-1,166,747 décrit des compositions moussantes contenant des savons et se présentant sous forme de crèmes onctueuses et confortables. Bien que ces compositions présentent une bonne stabilité physique à température ambiante et même jusqu'à au moins 45°C, il peut apparaître des grains macroscopiques dans ces crèmes quand on les stocke à une température bien supérieure à 45°C, par exemple 55°C, puis qu'on les refroidit à température ambiante (environ 25°C).

Par ailleurs, les compositions décrites dans EP-1,486,558, qui contiennent une certaine proportion d'acides gras ont une texture granuleuse quand on les ramène à température ambiante après les avoir chauffées à 55°C, comme le montrent les exemples comparatifs présentés plus loin.

Or, il est intéressant de disposer de crèmes qui soient stables même après passage à une température élevée, notamment dans les pays chauds ou aux saisons chaudes dans les pays tempérés. Il subsiste donc le besoin d'une composition moussante qui reste homogène et stable même après stockage à une température élevée, notamment à des températures d'au moins 50°C et même 55 °C.

La demanderesse a découvert de manière surprenante qu'il était possible d'améliorer la stabilité des produits à base de savons en ajoutant un tensioactif non ionique dérivé d'acide ou d'alcool en C22 (acide ou alcool béhénylique). Les compositions obtenues ne présentent pas de déphasage, ni de formation de grains après stockage à température élevée (55°C), et retour à la température ambiante.

L'association de ce type de tensioactif et de savons permet donc d'obtenir une composition très stable, et qui donne une mousse ayant de bonnes qualités cosmétiques et de bonnes performances de mousse.

Ainsi, la présente demande a pour objet une composition moussante pour application topique, contenant dans un milieu aqueux, un ou plusieurs sels d'acide gras en une quantité d'au moins 20 % du poids total de la composition, et au moins un tensioactif non ionique choisi parmi les dérivés d'alcool béhénylique, les dérivés d'acide béhénique et leurs mélanges.

La composition de l'invention peut se présenter sous forme de crèmes fluides à épaisses. Une crème est un produit souple par opposition à un produit solide tel qu'un stick, et elle a de préférence une viscosité allant de 3 à 200 poises (0,3 à 20 Pa.s), cette viscosité étant mesurée à la température ambiante (environ 25°C) à l'aide d'un Rhéomat RM180, 10 minutes après la mise de rotation du mobile, à une vitesse de 200 RPM, le mobile étant adapté à la viscosité.

La composition selon l'invention étant destinée à une application topique, elle contient un milieu physiologiquement acceptable. On entend par « milieu physiologiquement acceptable » dans la présente invention, un milieu non toxique, compatible avec la peau (y compris l'intérieur des paupières), les muqueuses, les cheveux ou les lèvres d'êtres humains. Cette composition peut constituer une composition cosmétique ou dermatologique, et notamment une composition cosmétique de nettoyage de la peau.

La composition selon l'invention a un pH compatible avec une application topique, et notamment avec une application sur la peau. Son pH va de préférence de 8 à 9,8 et mieux de 8,5 à 9,6.

### Sel d'acide gras

Le sel d'acide gras ou savon est obtenu à partir d'un acide gras et d'une base, l'acide gras comportant une chaîne alkyle linéaire ou ramifiée, saturée ou insaturée, ayant de 12 à 22 atomes de carbone et de préférence 12 à 20 atomes de carbone.

Les bases (aussi appelées aussi agents de saponification) neutralisent totalement ou partiellement les acides gras. Les bases susceptibles d'être utilisées pour obtenir les sels peuvent être par exemple les bases minérales comme les hydroxydes de métaux alcalins (hydroxyde de sodium et potasse), les hydroxydes de métaux alcalino-terreux (de magnésium) ou l'hydroxyde d'ammonium, ou encore les bases organiques comme la triéthanolamine, la N-méthylglucamine, la lysine et l'arginine. Selon un mode particulier de réalisation de l'invention, la base est la potasse.

Le savon est généralement introduit dans la composition sous forme de la base d'une part et de l'acide gras d'autre part, la formation du sel se faisant in situ.

Le taux de neutralisation de l'acide gras est défini comme étant le rapport massique entre les acides gras sous forme de sels et les acides gras totaux (acides libres + sels d'acides gras). Dans la composition selon l'invention, ce taux va de préférence de 80 à 97 %, et mieux de 85 à 95 %.

L'acide gras peut être choisi en particulier parmi les acides gras en C10 à C24, et notamment en C12-C18, et en particulier l'acide laurique, l'acide myristique, l'acide stéarique, l'acide palmitique et leurs mélanges.

Comme savons, on peut citer par exemple les sels de potassium et les sels de sodium des acides gras en C10 à C24, notamment en C12-C20, plus spécialement en C12-C18. Le savon peut être plus spécialement choisi parmi les sels de potassium des acides gras en C12-C18, plus spécialement le sel de potassium de l'acide laurique, le sel de potassium de l'acide myristique, le sel de potassium de l'acide palmitique, le sel de potassium de l'acide stéarique, et leurs mélanges.

La quantité totale de sels d'acide gras dans la composition de l'invention est d'au moins 20 % du poids total de la composition. Elle peut aller par exemple de 20 à 40 % en poids, de préférence de 25 à 40 % en poids et mieux de 25 à 35 % en poids par rapport au poids total de la composition.

Selon un mode préféré de réalisation de l'invention, la composition contient une quantité de sels d'acide gras en C12-C14, notamment d'acide laurique et d'acide myristique, d'au moins 10 % en poids et mieux d'au moins 15 % en poids par rapport au poids total de la composition.

### Dérivés d'alcool béhénylique et dérivés d'acide béhénique

L'acide béhénique et l'alcool béhénylique comportent 22 atomes de carbone. Le tensioactif non ionique dérivé de cet acide ou de cet alcool en C22 peut être notamment un dérivé comportant une partie polaire choisie parmi les motifs oxyéthylénés, les motifs oxyéthylénés et oxypropylénés, les motifs polyglycérolés, les motifs de sucre tel que le glucose, et leurs mélanges. Selon un mode préféré de réalisation de l'invention, le tensioactif non ionique en C22 est choisi parmi les dérivés oxyéthylénés de l'acide béhénique ou l'alcool béhénylique, notamment ceux comportant de 5 à 100 groupes oxyéthylénés.

De préférence, le tensioactif non ionique en C22 est choisi parmi les dérivés oxyéthylénés de l'alcool béhénylique, comportant de 5 à 100 groupes oxyéthylénés, et de préférence de 5 à 50 groupes oxyéthylénés, tels que le Beheneth-10 comme le produit commercialisé sous la dénomination EUMULGIN BA 10 par la société Cognis ou sous la dénomination EMALEX BHA-10 par la société Nihon Emulsion, ou le Béhéneth-20 comme le produit commercialisé sous la dénomination NIKKOL BB 20 par la société Nikko Chemicals. Selon un mode préféré de l'invention, le tensioactif non ionique choisi parmi les dérivés d'alcool béhénylique ou d'acide béhénique est choisi parmi le Beheneth-1 0 (dérivé d'alcool béhénylique avec 10 motifs oxyéthylénés), le Beheneth-20 (dérivé d'alcool béhénylique avec 20 motifs oxyéthylénés), et leurs mélanges.

La quantité de tensioactif non ionique choisi parmi les dérivés d'alcool béhénylique ou d'acide béhénique peut aller par exemple de 0,5 à 10 % en poids et de préférence de 1 à 5 % en poids par rapport au poids total de la composition.

### Autres tensioactifs

La composition selon l'invention peut en outre contenir un ou plusieurs tensioactifs additionnels, c'est-à-dire autres que les sels d'acide gras et les dérivés d'acide béhénique ou d'alcool béhénylique. Ces tensioactifs additionnels peuvent être choisis parmi les tensioactifs non ioniques, les tensioactifs amphotères ou zwitterioniques, les tensioactifs anioniques, les tensioactifs cationiques, et leurs mélanges. Quand ils sont présents, leur concentration peut aller de 0,1 % à 20 % du poids total de la composition et de préférence de 0,1 % à 10 % du poids total de la composition.

On peut utiliser par exemple comme tensioactifs non ioniques, les alkyl polyglucosides (APG), les esters de maltose, les alcools gras polyglycérolés, les dérivés de glucamine comme l'éthyl-2 hexyl oxy-carbonyl n-méthyl glucamine, et leurs mélanges.

Comme alkylpolyglucosides, on utilise de préférence ceux contenant un groupe alkyle comportant de 6 à 30 atomes de carbone et de préférence de 8 à 16 atomes de carbone, et contenant un groupe hydrophile (glucoside) comprenant de préférence 1,2 à 3 unités de saccharide. Comme alkylpolyglucosides, on peut citer par exemple le decylglucoside (Alkyl-C9/C11-polyglucoside (1.4)) comme le produit commercialisé sous la dénomination MYDOL 10® par la société Kao Chemicals, le produit commercialisé sous la dénomination PLANTAREN 2000 UP® par la société Cognis, et le produit commercialisé sous la dénomination ORAMIX NS 10® par la société Seppic ; le caprylyl/capryl glucoside comme le produit commercialisé sous la dénomination ORAMIX CG 110® par la Société Seppic ; le laurylglucoside comme les produits commercialisés sous les dénominations PLANTAREN 1200 N® et PLANTACARE 1200® par la société Cognis ; et le coco-glucoside comme le produit commercialisé sous la dénomination PLANTACARE 818/UP® par la société Cognis.

Les dérivés de maltose sont par exemple ceux décrits dans le document EP-A-566438, tels que l'O-octanoyl-6'-D-maltose, ou encore le O-dodecanoyl-6'-D-maltose décrit dans le document FR-2,739,556.

Parmi les alcools gras polyglycérolés on peut citer le dodecanediol polyglycérolé (3,5 moles de glycérol), produit commercialisé sous la dénomination CHIMEXANE NF® par la société Chimex.

Les tensioactifs amphotères et zwitterioniques peuvent être choisis par exemple parmi les dérivés de bétaïnes, les amidopropylbétaïnes, les amphoacétates, les hydroxylsultaines, et leurs mélanges.

Comme dérivés de bétaïnes, on peut citer par exemple la cocobétaïne comme le produit commercialisé sous la dénomination DEHYTON AB-30® par la société Cognis, la laurylbétaïne comme le produit commercialisé sous la dénomination GENAGEN KB® par la société Clariant, la laurylbétaïne oxyethylénée (10 OE), comme le produit commercialisé sous la dénomination LAURYLETHER(10 OE)BETAINE® par la société Shin Nihon Rica, la stéarylbétaïne oxyéthylénée (10 OE) comme le produit commercialisé sous la dénomination STEARYLETHER(10 OE)BETAINE® par la société Shin Nihon Rica, la cocamidopropyl betaine commercialisé par exemple sous la dénomination VELVETEX BK 35® par Cognis ou encore le undecylenamidopropyl betaïne commercialisé par exemple sous la dénomination AMPHORAM U® par Ceca.

Comme alkylamphoacétates, on peut citer par exemple le N-cocoyl-N-carboxyméthoxyéthyl-N-carboxyméthyl-éthylènediamine N-di-sodique (nom INCI: disodium cocamphodiacetate) comme le produit commercialisé sous la dénomination MIRANOL C2M CONCENTRE NP® par la société Rhodia Chimie, et le N-cocoyl-N-hydroxyéthyl-N-carboxyméthyl-éthylènediamine N-sodique (nom INCI : sodium cocamphoacetate).

Les tensioactifs anioniques peuvent être choisis par exemple parmi les sarcosinates et acylsarcosinates et leurs sels tels que le lauroyl sarcosinate de sodium ; les taurates et méthyltaurates et leurs sels; les iséthionates et acyl iséthionates, produits de réaction d'acides gras comportant de 10 à 22 atomes de carbone, avec l'acide isethionique, et leurs sels tels que l'iséthionate de sodium et le cocoyl-iséthionate de sodium ; les sulfosuccinates et leurs sels ; les alkylsulfates et alkyléthersulfates et leurs sels, notamment le laurylsulfate de sodium ou de triéthanolamine, et le lauryléther sulfate de sodium ou de potassium ; les monoalkyl- et dialkylesters d'acide phosphorique et leurs sels, tels que par exemple le mono- et dilauryl phosphate de sodium, le mono- et dilauryl phosphate de potassium, le mono- et dilauryl phosphate de triéthanolamine, le mono- et dimyristyl phosphate de sodium, le mono- et dimyristyl phosphate de potassium, le mono- et dimyristyl phosphate de diéthanolamine, le mono- et dimyristyl phosphate de triéthanolamine ; les alkane sulfonates et leurs sels ; les sels biliaires tels que les cholates, déoxycholates, taurocholates, taurodéoxycholates ; les lipoaminoacides et leurs sels tels que les acylglutamates mono- et disodiques ; et leurs mélanges.

Selon un mode particulier de réalisation de l'invention, la composition contient au moins un tensioactif non ionique choisi parmi les alkyl polyglucosides, comme tensioactif additionnel.

### Additifs

Le milieu aqueux de la composition moussante de l'invention étant destiné à une application topique est un milieu physiologiquement acceptable, et il contient de l'eau qui peut représente au moins 10 % du poids total de la composition, et de préférence au moins 20 % du poids total de la composition. La quantité d'eau peut aller par exemple de 10 à 75 % en poids, de préférence de 20 à 70 % en poids et mieux de 20 à 60 % en poids par rapport au poids total de la composition.

La composition peut contenir, outre l'eau, un ou plusieurs solvants choisis parmi les alcools inférieurs comportant de 1 à 6 atomes de carbone, tels que l'éthanol ; les polyols tels que la glycérine ; les glycols comme le butylène glycol, l'isoprène glycol, le propylène glycol, les polyéthylène glycols tels que le PEG-8 ; le sorbitol ; les sucres tels que le glucose, le fructose, le maltose, le lactose, le sucrose ; et leurs mélanges. La quantité de solvant(s) dans la composition de l'invention peut aller de 0,5 à 50 % en poids et de préférence de 5 à 40 % en poids par rapport au poids total de la composition.

Selon un mode préféré de réalisation de l'invention, la composition contient un ou plusieurs polyols, et de préférence au moins 5 % en poids d'un ou plusieurs polyols par rapport au poids total de la composition.

La composition de l'invention peut comprendre aussi tous les additifs ou actifs classiquement utilisés dans les produits pour application topique, notamment cosmétique ou dermatologique. On peut citer par exemple les adjuvants hydrosolubles ou liposolubles habituels dans le domaine cosmétique ou dermatologique, tels que les conservateurs, les séquestrants (EDTA), les antioxydants, les parfums, les matières colorantes, les colorants solubles ou les pigments encapsulés ou non, les nacres, des charges (charges à effets matifiant, tenseur, blanchissant ou exfoliant), les filtres solaires, les polymères, les actifs cosmétiques ou dermatologiques, hydrophiles ou lipophiles.

Les quantités de ces différents adjuvants sont celles classiquement utilisées dans le domaine considéré, et par exemple de 0,01 à 20 % du poids total de la composition. Ces adjuvants ainsi que leurs concentrations doivent être tels qu'ils ne modifient pas la propriété recherchée pour la composition de l'invention.

Comme charges qui peuvent être utilisées dans la composition de l'invention, on peut citer par exemple, la silice et en particulier les microbilles de silice telles que celles commercialisées sous la dénomination SB150 par la société Myoshi ; le talc ; le kaolin ; les billes de verre et de céramique commercialisées par la société 3M sous la dénomination commerciale MACROLITE ; les particules de polyamide et notamment celles vendues sous les dénominations ORGASOL par la société Atochem ; les poudres de polymères synthétiques non expansées, telles que les poudres de polyéthylène, de polystyrène, de polyesters, de polyamides (par exemple le nylon ou la poly-β-alanine), de copolymères d'acrylates (par exemple les microsphères microporeuses vendues par la société Dow Corning sous la dénomination commerciale POLYTRAP), d'acides polyméthacryliques, de Téflon^{®} (polytétrafluoroéthylène ou PTFE) comme les produits commercialisés sous les dénominations FLUON par la société Uniqema et le produit commercialisé sous la dénomination FLUOROPURE 812C par la société Shamrock (poudre de PTFE ayant une taille des particules 12 µm); les poudres expansées telles que les microsphères creuses en matériau thermoplastique préparées par des procédés connus, comme ceux décrits dans les documents US-A-3 615 972 et EP-A-0 56219, et notamment, les microsphères commercialisées sous les dénominations EXPANCEL par la société Kemanord Plast ou sous la dénomination MICROPEARL F 80 ED par la société Matsumoto ; les poudres de matériaux organiques naturels tels que les poudres d'amidons (de maïs, de blé ou de riz,) réticulés ou non, modifiés ou non, telles que les poudres d'amidon réticulé par l'anhydride octénylsuccinate, commercialisées sous la dénomination DRY-FLO par la société National Starch ; les microbilles de résine de silicone telles que celles commercialisées sous la dénomination TOSPEARL par la société Toshiba Silicone ; et leurs mélanges.

Comme actifs, on peut citer par exemple les vitamines hydrosolubles ou liposolubles, telles que la vitamine A (rétinol), la vitamine C (acide ascorbique), la vitamine E (tocophérol), la vitamine B3 (ou vitamine PP ou niacinamide), la vitamine B5 (panthénol sous ses différentes formes : D-panthénol, DL-panthénol), la vitamine D, la vitamine F (mélange d'acides gras essentiels), les dérivés, précurseurs et analogues de ces vitamines ; les α-hydroxy-acides notamment les acides dérivés de fruits, comme les acides glycolique, lactique, malique, citrique, tartrique, mandélique, leurs dérivés et leurs mélanges ; les β-hydroxy-acides comme l'acide salicylique et ses dérivés tels que l'acide n-octanoyl-5-salicylique ou l'acide n-dodécanoyl-5 salicylique ; les antiseptiques ; les antiséborrhéïques et antimicrobiens tels que le peroxyde de benzoyle, l'acide salicylique, le 2,4,4'-trichloro-2'-hydroxy diphényl éther (ou triclosan), le 3,4,4'-trichlorocarbanilide (ou triclocarban), l'acide azélaïque, les sels de zinc tels que le gluconate de zinc ; les amincissants tels que la caféine ; les azurants optiques ; les électrolytes ; et de manière générale tout agent ayant pour effet d'améliorer les propriétés cosmétiques de la peau.

Les polymères utilisables dans la composition de l'invention peuvent être hydrophiles ou lipophiles, anioniques, non ioniques, cationiques ou amphotères, épaississants ou dispersants:

Comme polymères, on peut citer par exemple les polysaccharides comme la gomme de xanthane, la gomme de guar, les alginates, les celluloses modifiées ; les polymères synthétiques tels que les polymères et copolymères acryliques, les polymères et copolymères dérivés d'acrylamides, les polyalkylène glycols, de préférence les polyéthylène glycols, de poids moléculaire élevé, et les mélanges de ces polymères.

Les polyéthylène glycols de poids moléculaire élevé ont un nombre de motifs d'oxyde d'éthylène (OE) supérieur à 10, de préférence d'au moins 50 et mieux d'au moins 100, comme le polyéthylène glycol comportant 14000 OE (nom CTFA : PEG-14 M), le polyéthylène glycol comportant 7000 OE (nom CTFA : PEG-7M), le polyéthylène glycol comportant 20000 OE (nom CTFA : PEG-20M), le polyéthylène glycol comportant 60000 OE (nom CTFA : PEG-60 M).

On peut citer aussi comme polymères, les polymères cationiques du type polyquaternium, qui apportent douceur et onctuosité à la composition moussante. Ces polymères cationiques peuvent être choisis de préférence parmi les polymères suivants :
- Polyquaternium 5 tel que le produit MERQUAT 5 commercialisé par la société CALGON ;
- Polyquaternium 6 tel que le produit SALCARE SC 30 commercialisé par la société CIBA, et le produit MERQUAT 100 commercialisé par la société CALGON ;
- Polyquaternium 7 tel que les produits MERQUAT S, MERQUAT 2200 et MERQUAT 550 commercialisés par la société CALGON, et le produit SALCARE SC 10 commercialisé par la société CIBA;
- Polyquaternium 10 tel que le produit Polymer JR400 commercialisé par la société AMERCHOL ;
- Polyquaternium 11 tel que les produits GAFQUAT 755, GAFQUAT 755N et GAFQUAT 734 commercialisés par la société ISP ;
- Polyquaternium 15 tel que le produit ROHAGIT KF 720 F commercialisé par la société ROHM ;
- Polyquaternium 16 tel que les produits LUVIQUAT FC905, LUVIQUAT FC370, LUVIQUAT HM552 et LUVIQUAT FC550 commercialisés par la société BASF ;
- Polyquaternium 22 tel que le produit MERQUAT 280 commercialisé par la société CALGON ;
- Polyquaternium 28 tel que le produit STYLEZE CC10 commercialisé par la société ISP ;
- Polyquaternium 39 tel que le produit MERQUAT PLUS 3330 commercialisé par la société CALGON ;
- Polyquaternium 44 tel que le produit LUVIQUAT CARE commercialisé par la société BASF ;
- Polyquaternium 46 tel que le produit LUVIQUAT HOLD commercialisé par la société BASF ;
- Polyquaternium 47 tel que le produit MERQUAT 2001 commercialisé par la société CALGON.

On peut aussi utiliser comme polymère cationique les guars cationiques telles que le produit JAGUAR commercialisé par la société RHODIA.

Les compositions selon l'invention peuvent constituer notamment des produits de nettoyage ou de démaquillage de la peau (corps ou visage y compris yeux), du cuir chevelu et/ou des cheveux. Elles peuvent constituer plus particulièrement une composition de nettoyage de la peau.

Ces compositions développent de la mousse quand elles sont mélangées avec de l'eau. Elles peuvent être utilisées de deux façons :
- la première utilisation consiste à étaler la composition dans les mains, à l'appliquer sur le visage ou sur le corps puis à la masser en présence d'eau pour développer la mousse directement sur le visage ou le corps.
- l'autre utilisation possible de cette composition consiste à développer la mousse dans les paumes des mains avant d'appliquer la mousse obtenue sur le visage ou le corps.

Dans les deux cas, la mousse est ensuite rincée.

Un autre objet de l'invention consiste en l'utilisation cosmétique de la composition telle que définie ci-dessus, comme produit de nettoyage et/ou de démaquillage de la peau, du cuir chevelu et/ou des cheveux.

Un autre objet de l'invention consiste en un procédé cosmétique de nettoyage de la peau, du cuir chevelu et/ou des cheveux, caractérisé en ce qu'on applique la composition de l'invention, sur la peau, sur le cuir chevelu et/ou sur les cheveux, en présence d'eau, et qu'on élimine la mousse formée et les résidus de salissure par rinçage à l'eau.

Les exemples qui suivent servent à illustrer l'invention sans toutefois présenter un caractère limitatif. Les quantités indiquées sont en % en poids sauf mention contraire.

| | Exemple 1 | Exemple comparatif 1 | Exemple comparatif 2 | Exemple comparatif 3 |
|---|---|---|---|---|
| Acide laurique | 3 | 3 | 3 | 3 |
| Acide myristique | 20 | 20 | 20 | 20 |
| Acide stéarique | 6 | 6 | 6 | 6 |
| Glycéryl stéarate | 2 | 2 | 2 | 2 |
| Decyl glucoside | 1 | 1 | 1 | 1 |
| Potasse | 6.2 | 6.2 | 6.2 | 6.2 |
| Glycérine | 14 | 14 | 14 | 14 |
| PEG 8 | 7 | 7 | 7 | 7 |
| Beheneth-10 | 1 | - | - | - |
| Acide béhénique | - | - | 1 | - |
| Alcool béhénylique | - | - | - | 1 |
| Conservateurs | 0.9 | 0.9 | 0.9 | 0.9 |
| Eau | Qsp 100 | Qsp 100 | Qsp 100 | Qsp 100 |
| pH | 9.3 | 9.3 | 9.4 | 9.3 |
| Aspect après stockage pendant 2 mois à chaud (à 55°C) et retour à la température ambiante | Texture homogène | Granuleux | Granuleux | Granuleux |

Dans ces exemples, le taux de neutralisation des acides gras est d'environ 90 %.

### Mode opératoire :

Les acides gras, le beheneth-10 et le glycéryl stéarate ont été dispersés dans la phase aqueuse comprenant l'eau, les polyols et les conservateurs, à l'aide d'une turbine rotor-stator. Ils ont été ensuite neutralisés à la potasse à 80°C. Le mélange a été lentement refroidi sous agitation avec les pâles, et le décyl glucoside a été ajouté vers 40°C. Le mélange a ensuite été brassé à la pâle jusqu'à refroidissement complet à 25°C.

Les exemples comparatifs 1 à 3 présentent un aspect granuleux après le stockage à une température de 55°C. Ces exemples comparatifs montrent que le remplacement du tensioactif dérivé d'alcool béhénylique par l'acide béhénique ou l'alcool béhénylique ne permet pas d'atteindre le but de l'invention.

## Revendications

1. Composition moussante pour application topique, contenant dans un milieu aqueux, un ou plusieurs sels d'acide gras en une quantité d'au moins 20 % du poids total de la composition, et au moins un tensioactif non ionique choisi parmi les dérivés d'alcool béhénylique, les dérivés d'acide béhénique et leurs mélanges.

2. Composition selon la revendication 1, **caractérisée en ce que** le pH va de 8 à 9,8.

3. Composition selon la revendication 1 ou 2, **caractérisée en ce que** le taux de neutralisation de l'acide gras va de 80 à 97 %.

4. Composition selon l'une quelconque des revendications précédentes, **caractérisée en ce que** l'acide gras est choisi parmi l'acide laurique, l'acide myristique, l'acide stéarique, l'acide palmitique et leurs mélanges.

5. Composition selon l'une quelconque des revendications précédentes, **caractérisée en ce que** le sel d'acide gras est choisi parmi les sels de potassium des acides gras en C12-C18.

6. Composition selon l'une quelconque des revendications précédentes, **caractérisée en ce que** la quantité totale de sels d'acide gras va de 20 à 40 % en poids par rapport au poids total de la composition.

7. Composition selon l'une quelconque des revendications précédentes, **caractérisée en ce qu'**elle contient une quantité de sels d'acide gras en C12-C14 d'au moins 10 % en poids par rapport au poids total de la composition.

8. Composition selon l'une quelconque des revendications précédentes, **caractérisée en ce que** le tensioactif non ionique choisi parmi les dérivés d'alcool béhénylique et les dérivés d'acide béhénique est un dérivé comportant une partie polaire choisie parmi les motifs oxyéthylénés, les motifs oxyéthylénés et oxypropylénés les motifs polyglycérolés, les motifs de sucre, et leurs mélanges.

9. Composition selon la revendication précédente, **caractérisée en ce que** les dérivés d'alcool béhénylique et les dérivés d'acide béhénique sont des dérivés oxyéthylénés comportant de 5 à 100 groupes oxyéthylénés.

10. Composition selon l'une quelconque des revendications précédentes, **caractérisée en ce que** le tensioactif non ionique est choisi parmi les dérivés oxyéthylénés d'alcool béhénylique, comportant de 5 à 100 groupes oxyéthylénés.

11. Composition selon l'une quelconque des revendications précédentes, **caractérisée en ce** la quantité de tensioactif non ionique choisi parmi les dérivés d'alcool béhénylique et les dérivés d'acide béhénique va de 0,5 à 10 % en poids par rapport au poids total de la composition.

12. Composition selon l'une quelconque des revendications précédentes, **caractérisée en ce qu'**elle comprend en outre, un ou plusieurs tensioactifs additionnels choisis parmi les tensioactifs non-ioniques, les tensioactifs amphotères ou zwitterioniques, les tensioactifs anioniques, les tensioactifs cationiques, et leurs mélanges.

13. Composition selon la revendication précédente, **caractérisée en ce que** le tensioactif additionnel est un alkyl polyglucoside.

14. Composition selon la revendication 12 et 13, **caractérisée en ce que** la quantité de tensioactifs additionnels va de 0,1 à 20 % en poids par rapport au poids total de la composition.

15. Composition selon l'une quelconque des revendications précédentes, **caractérisée en ce qu'**elle constitue une composition de nettoyage de la peau.

16. Utilisation cosmétique d'une composition selon l'une quelconque des revendications 1 à 14, comme produit de nettoyage et/ou de démaquillage de la peau, du cuir chevelu et/ou des cheveux.
